# EUROPEAN PATENT SPECIFICATION

(11) **EP 4 387 575 B1**
(45) Date of publication and mention of the grant of the patent: **23.09.2026**
(21) Application number: 22859194.7
(22) Date of filing: 19.08.2022
(51) Int. Cl.: A61F 13/00, A61F 13/02

(54) **MULTI-LAYER DRESSING COVER FOR PROVIDING ACCESS TO PATIENT IMPLANTS AND WOUNDS**
MEHRSCHICHTIGE VERBANDSABDECKUNG ZUR BEREITSTELLUNG DES ZUGANGS ZU PATIENTENIMPLANTATEN UND WUNDEN
REVÊTEMENT DE PANSEMENT MULTICOUCHE POUR FOURNIR UN ACCÈS À DES IMPLANTS ET DES PLAIES DE PATIENT

(30) Priority: 19.08.2021 US 202163234991 P
(43) Date of publication of application: 26.06.2024
(73) Proprietor: Becton, Dickinson and Company, Franklin Lakes, NJ 07417 (US)
(72) Inventor: RYAN, Kevin, M., Whitehouse Station, New Jersey 08889 (US)
(74) Representative: dompatent
(86) International application number: PCT/US2022/040832
(87) International publication number: WO 2023/023294

(56) References cited:
- US-A- 5 380 294
- US-A1- 2002 169 405
- US-A1- 2013 102 945
- US-A1- 2016 193 452
- US-A1- 2017 368 310
- US-A1- 2018 050 175
- US-A1- 2018 161 543
- US-A1- 2018 161 543
- US-B2- 10 456 497

## Description

### CROSS-REFERNCE TO RELATED APPLICATION

The present application claims priority to United States Provisional Application No. 63/234,991, filed August 19, 2021, entitled "Multi-Layer Dressing Cover for Providing Access to Patient Implants and Wounds'.

### BACKGROUND OF THE INVENTION

### Field of the Invention

The present disclosure relates to dressing covers, and more particularly to dressing covers having multiple layers.

### Description of Related Art

Intravenous (IV) therapy is a medical technique used to deliver fluids, medications, and/or nutrition directly into a body of a patient via an IV line (e.g., an IV cannula, an IV catheter, etc.) inserted into a vein. IV therapy may be used for rehydration, to provide nutrition to a person who cannot consume food or water by mouth, and/or to administer medications or other medical therapy, such as blood products or electrolytes. IV lines are conventionally held to the insertion site in the patient by dressing covers that have an adhesive which bonds to the patient's skin surrounding the insertion site.

Various adhesives may be used to facilitate bonding of the dressing cover to the patient's skin. Generally, it may be desirable to minimize movement of the IV line once attached to the patient. Movement of the IV line can cause irritation of a blood vessel, disrupt proper introduction of medications to the patient, and/or increase the potential for bleeding or infection at the IV insertion site. If excessive movement occurs, the IV catheter can even come out of the patient, interrupting delivery of medication and requiring re-insertion of the catheter. Accordingly, a strong adhesion force between the dressing cover and the patient's skin may be desired to ensure secure positioning of the IV catheter during use.

Conversely, a weaker adhesion force may be desirable if removal of the dressing cover is anticipated. For example, a clinician may need periodic access to the insertion site for inspection, cleaning, to administer treatment, etc. On such occasions, the clinician will typically remove the dressing cover from the patient, perform the required task, and reattach (or replace) the dressing cover. Removal of the dressing cover can cause pain and skin injury as the adhesive is separated from the patient's skin. Accordingly, a weaker adhesion force between the dressing cover and the patient's skin may be desired to reduce patient discomfort and the risk of skin injury.

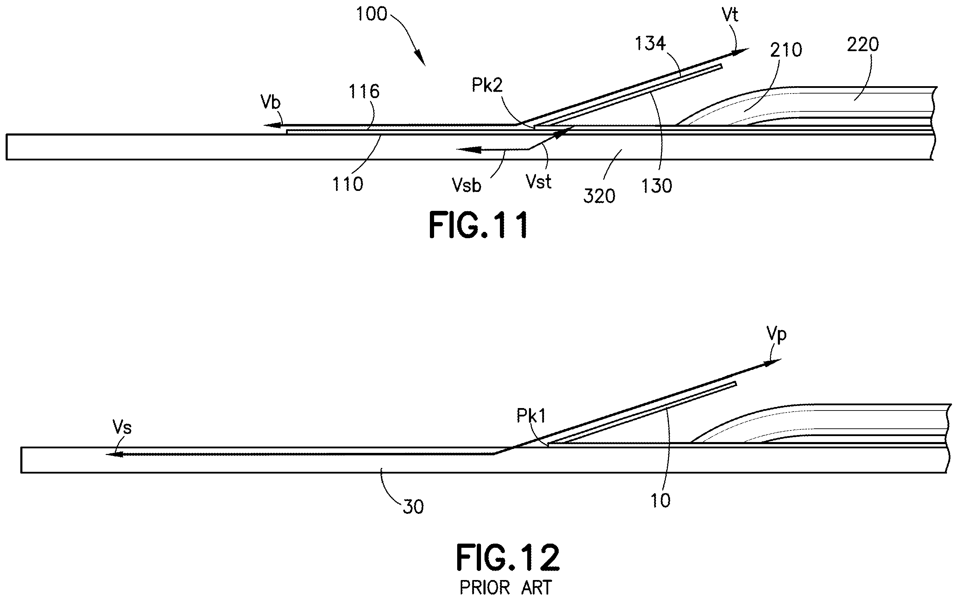

Conventional dressing covers balance the desire to securely hold the IV line to the patient with the competing desire reduce patient discomfort and risk of injury during removal of the dressing cover. An example of a dressing cover of the prior art is shown in FIG. 12, which illustrates the forces applied to the patient's skin during removal of the dressing cover. Referring to FIG. 12, as the dressing cover 10 is peeled away from the patient's skin 30, a tension force, illustrated by vector Vp, is developed in the dressing cover 10. The tension force may correspond to the force required to separate the adhesive of the dressing cover 10 from the patient's skin 30. The tension force developed in the dressing cover 10 is transferred to the patient's skin 30, illustrated by vector Vs. Substantially all of the tension force applied to the dressing cover 10 may be imparted to the patient's skin 30 (i.e. the magnitudes of vector Vp and vector Vs may be substantially equal), creating a significant risk of pain and/or skin injury during removal of the dressing cover 10. Further, a stress concentration occurs at a point Pk1 on the patient's skin 30 where the dressing cover 10 separates from the patient's skin 30. The stress concentration further exacerbates the risk and severity of pain and/or skin injury during removal of the dressing cover 10.
US 2018/161543 A1 discloses catheter securement dressings that aid in applying skin adhesive to a catheter insertion site. Catheter securement dressings can include a first dressing layer that secures a position of a catheter at a catheter insertion site following catheterization. The first dressing layer can include an access window that provides access to the catheter insertion site to allow for application of skin adhesive to the catheter insertion site. After the skin adhesive has dried, a second dressing layer can be folded over the first dressing layer to cover the access window.
US 2017/368310 A1 discloses: A dressing having a base which is to be adhered to a patient about a catheter site, an aperture in the base to pull a catheter line through and a cover with a window that is folded over and onto the base in a sealed manner.
US 2002/169405 A1 discloses: A self adherent dermal wound window dressing includes a fabric tape layer having an adhesive skin adhering side and an opposite non-adhesive side. The fabric tape layer has an opening therein to allow viewing therethrough. A semipermeable transparent film layer closes the opening in the fabric tape layer and has an adhesive skin adhering side and an opposite non-adhesive side. The film layer non-adhesive side is adhered on the adhesive side of the fabric layer around the opening such that the fabric layer extends beyond the periphery of the transparent film layer. An absorbent fiber layer having an opening generally corresponding to the opening in the fabric tape layer, is mounted on the adhesive skin adhering side of the transparent film layer such that the openings in the absorbent fiber and fabric tape layers are in alignment and the transparent film layer extends beyond the periphery of the absorbent fiber layer.
US 2013/102945 A1 discloses: A light-weight, semi-rigid wound shield protecting wounds and intradermal therapy ports from impacts between therapy sessions. The wound shield comprises a domed cover forming a concave cavity above the wound or therapy port. A flange surrounding the cavity presses against the patient's skin to deflect and distribute the force of impacts. The flange may include a self-adhesive layer on its bottom, or it may be secured with medical tape or a fitted bandage that overlaps the flange and the surrounding skin. A peel-away membrane covers the adhesive layers. A tunnel through the flange may connect to the cavity to accommodate a therapy tube which remains in place while the shield is worn. In an alternate embodiment, a base coextensive with the flange adheres to the patient's skin and the wound shield removably couples to the base by its flange. The assembly comes in a sterile envelope or package.
US 10,456,497 B2 discloses a dressing for use in protectively covering and isolating a medical device placed on and/or through the skin surface of a patient is disclosed. Examples of such devices include infusion needles for accessing subcutaneously implanted access ports, catheters of various types and purposes, insulin infusion needles, etc. In one embodiment, a dressing for covering a medical device on a skin surface of a patient and comprises a dressing portion that is configured to rest against a skin surface of a patient, with the dressing portion defining a hole, and a polymeric cover film that is at least indirectly attached to the dressing portion. The cover film includes a pliable domed portion aligned with the hole of the dressing portion. The pliable domed portion defines a cavity that is configured to receive therein the medical device when the dressing is placed on the skin of the patient.

### SUMMARY

In view of the foregoing, there exists a need for dressing covers that can securely hold an IV catheter in place without causing patient discomfort and potential skin injury during removal of the dressing cover.

In accordance with an aspect of the present disclosure, a dressing cover according to claim 1 is disclosed. The dressing cover includes a base layer having an adhesive configured to adhere to skin of a patient, the base layer defining a cutout configured to provide access to a cannula insertion site of the skin. The dressing cover also includes a top layer having an adhesive removably adhered to the base layer, the top layer configured to cover the insertion site and including an at least partially transparent window overlaying the cutout of the base layer, the window configured to allow observation of the insertion site.

In some embodiments, the base layer extends beyond a perimeter of the top layer.

In some embodiments, the base layer includes an outwardly extending tab.

In some embodiments, the top layer includes an outwardly extending tab.

In some embodiments, the adhesive of the top layer is not present on the outwardly extending tab of the top layer.

In some embodiments, the adhesive of the top layer is weaker on the tab than on a remaining portion of the top layer.

In some embodiments, the base layer includes an outwardly extending tab, and the outwardly extending tab of the top layer overlaps the outwardly extending tab of the base layer.

According to the present invention, the top layer includes a tunnel configured to receive a treatment line therethrough.

According to the present invention, the tunnel terminates at an intermediate portion of the window.

In some embodiments, the adhesive of the top layer is not present on the tunnel.

In some embodiments, the top layer includes an outwardly extending tab extending from an opposite side of the top layer relative to the tunnel.

In some embodiments, the base layer includes an adhesive pad configured to adhere to the treatment line.

In some embodiments, the adhesive of the top layer is a static adhesive.

In some embodiments, the adhesive of the top layer is weaker than the adhesive of the base layer.

In some embodiments, the adhesive of the top layer is stronger than the adhesive of the base layer.

In accordance with another aspect of the present disclosure, a treatment line and dressing cover assembly are disclosed. The assembly includes a dressing cover as described before. The assembly also includes a treatment line secured between the base layer and the top layer, the treatment line having a cannula configured for insertion in the insertion site.

In some embodiments, the base layer extends beyond a perimeter of the top layer.

In some embodiments, the base layer includes an adhesive pad configured to adhere to the treatment line.

Further details and advantages of the various examples described in detail herein will become clear upon reviewing the following detailed description of the various examples in conjunction with the accompanying drawing figures.

### BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:
**FIG. 1** is a top perspective view of a dressing cover according to one aspect or embodiment of the present disclosure, shown securing an IV line to a patient;
**FIG. 2** is an exploded perspective view of the dressing cover of **FIG. 1****;**
**FIG. 3** is a top perspective view of the dressing cover of **FIG. 1****,** shown adhered to a patient;
**FIG. 4** is a top perspective view of the dressing cover of **FIG. 1****;**
**FIG. 5** is a top perspective view of a base layer of the dressing cover of **FIG. 1****;**
**FIG. 6** is a bottom perspective view of the base layer of **FIG. 5****;**
**FIG. 7** is a top perspective view of a top layer of the dressing cover of **FIG. 1****,** with a window thereof not shown for clarity;
**FIG. 8** is a bottom perspective view of the top layer of **FIG. 7****;**
**FIG. 9** is a top perspective view of a window of the top layer of **FIG. 7****;**
**FIG. 10** is a top perspective view of the dressing cover of **FIG. 1****,** with the top layer partially removed from the base layer;
**FIG. 11** is a side view of the dressing cover of **FIG. 1****,** with the top layer partially removed from the base layer; and
**FIG. 12** is a side view of a prior art dressing cover partially removed from the patient.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DETAILED DESCRIPTION

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary aspects of the invention. Hence, specific dimensions and other physical characteristics related to the aspects disclosed herein are not to be considered as limiting.

Unless otherwise indicated, all ranges or ratios disclosed herein are to be understood to encompass the beginning and ending values and any and all subranges or subratios subsumed therein. For example, a stated range or ratio of "1 to 10" should be considered to include any and all subranges or subratios between (and inclusive of) the minimum value of 1 and the maximum value of 10; that is, all subranges or subratios beginning with a minimum value of 1 or more and ending with a maximum value of 10 or less.

The terms "first", "second", and the like are not intended to refer to any particular order or chronology, but refer to different conditions, properties, or elements.

As used herein with reference to a device or apparatus connected to a patient, the term "proximal" refers to an end of the device or apparatus farthest from the patient, or to a direction toward the end of the device or apparatus farthest from the patient. As used herein with reference to a device or apparatus connected to a patient, the term "distal" refers to an end of the device or apparatus closest to the patient (e.g. the end of the device or apparatus connected to the patient), or to a direction toward the end of the device or apparatus closest to the patient.

As used herein, "at least one of" is synonymous with "one or more of". For example, the phrase "at least one of A, B, and C" means any one of A, B, or C, or any combination of any two or more of A, B, or C. For example, "at least one of A, B, and C" includes one or more of A alone; or one or more of B alone; or one or more of C alone; or one or more of A and one or more of B; or one or more of A and one or more of C; or one or more of B and one or more of C; or one or more of all of A, B, and C.

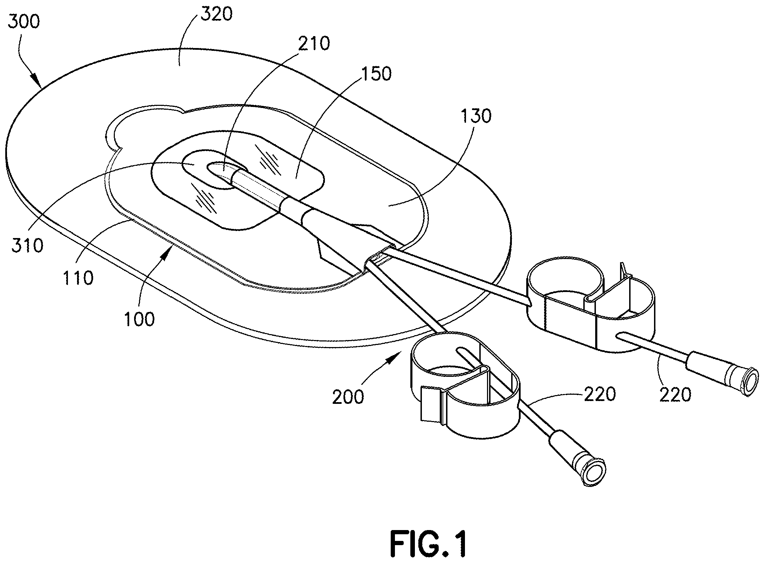

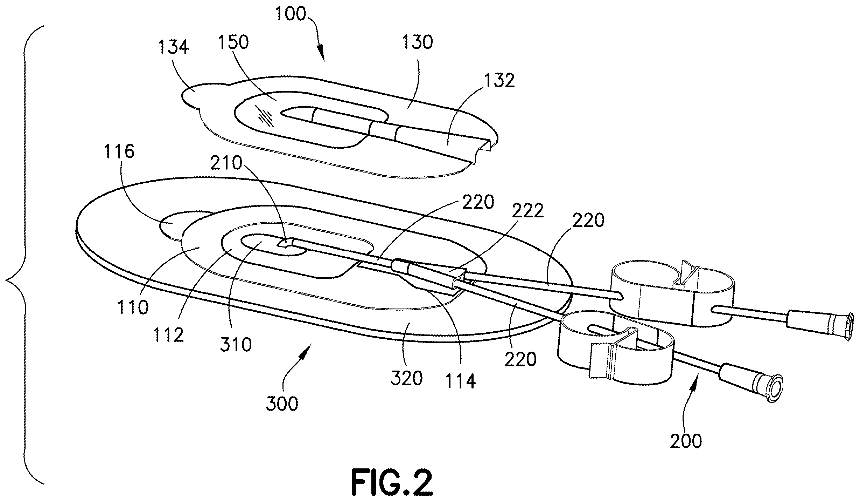

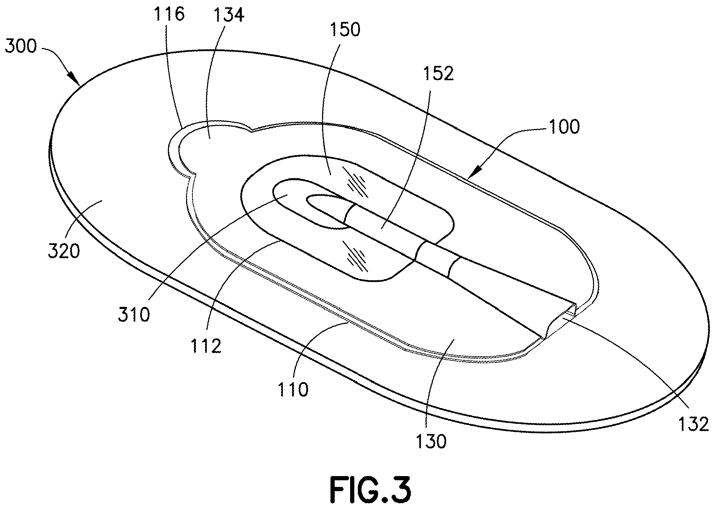

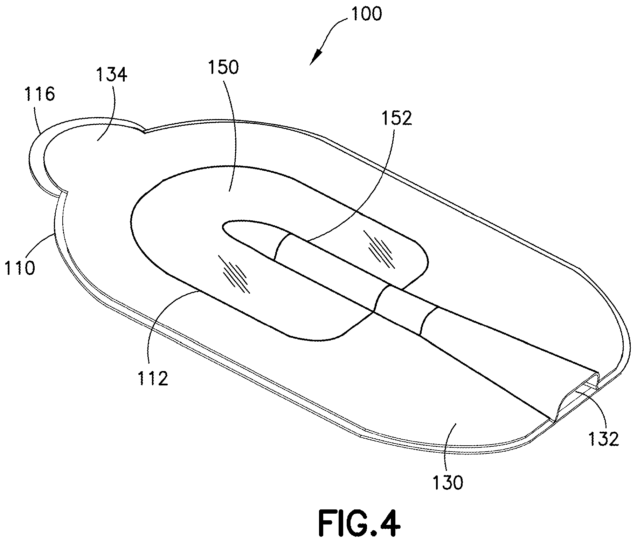

Non-limiting embodiments of the present disclosure are directed to a dressing cover for securing a treatment line, such as an IV treatment line, to a cannula insertion site of a patient's skin. The insertion site may correspond to a location of a vein into which a cannula or catheter of the IV treatment line is inserted to deliver fluid to the patient. Referring first to FIGS. 1-4, a dressing cover 100 is shown securing an IV treatment line 200 to an insertion site 310 of a patient 300. The dressing cover 100 may, in particular, be adhered to the patient's skin 320 surrounding the insertion site 310. The dressing cover 100 generally includes a base layer 110 having an adhesive configured for adhering to the patient's skin 320 and a top layer 130 removably adhered to the base layer 110. The IV treatment line 200 may be arranged between the base layer 110 and the top layer 130, such that the IV treatment line 200 is held in place relative to the insertion site 310 by the dressing cover 100. The top layer 130 covers and protects the insertion site 310 during administration of fluid to the patient 300 via the IV treatment line 200. The top layer 130 is removable from the base layer 110 to allow a clinician access to the insertion site 310 and the IV treatment line 200 without removing the base layer 110 from the patient's skin 320.

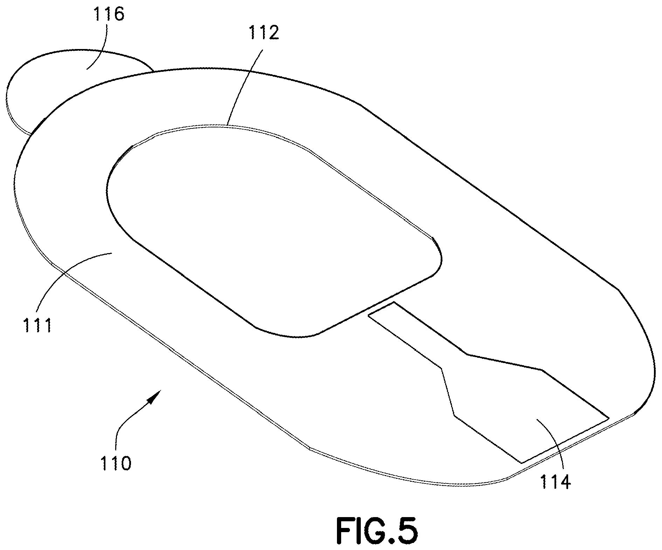

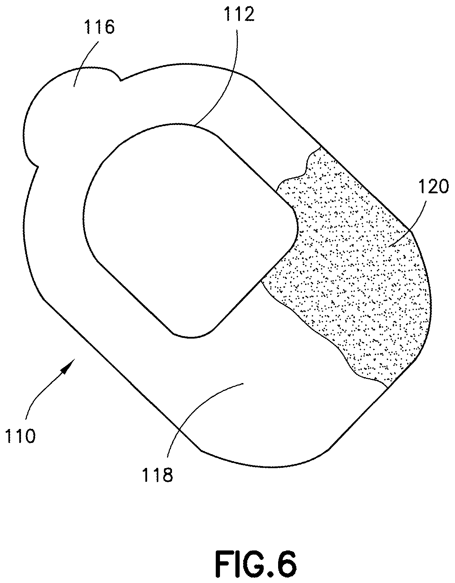

With continued reference to FIGS. 1-4 and further reference to FIGS. 5 and 6, the base layer 110 includes or define a cutout 112 providing access to the insertion site 310. In particular, the base layer 110 may be applied to the patient's skin 320 such that the cutout 112 surrounds the insertion site 310. The cutout 112 may be circular, elliptical, polygonal, or any other shape. The base layer 110 includes an adhesive pad 114 for receiving and holding the IV treatment line 200. In some non-limiting embodiments, the adhesive pad 114 may be positioned so as to receive a hub 222 of the IV treatment line 200. The hub 222 may be a junction where one or more lumens 220 of the IV treatment line 200 merge. The hub 222 may have a flat surface configured to adhere to the adhesive pad 114. In some non-limiting embodiments, the one or more lumens 220 may be adhered to the adhesive pads 114 to secure the IV treatment line 200 in place relative to the insertion site 310.

With continued reference to FIGS. 1-6, the base layer 110 may be of substantially any shape. In the non-limiting embodiments shown in the accompanying drawings, the base layer 110 may be four-sided with rounded corners. The cutout 112 may likewise be of substantially any shape, and is desirably large enough to provide ready view of and access to the insertion site 310. The base layer 110 may be made from a material having a higher tension strength (or higher stress vs. strain curve) than human skin, such that the base layer 110 mitigates forces applied to the patient's skin 320 during removal of the top layer 130. Further details of the role of the base layer 110 in mitigating forces on the patient's skin are described herein with reference to FIG. 11.

With continued reference to FIG. 6, the base layer 110 may include an outwardly extending tab 116. The tab 116 may be held against the patient's skin 320 by the clinician when the top layer 130 is removed to prevent the base layer 110 from inadvertently detaching from the patient's skin 320. The tab 116 may extend from the opposite side of the base layer 110 as the tunnel 132 (and thus opposite from the IV treatment line 200 extending through the tunnel 132) to reduce the likelihood of jostling the IV treatment line 200 when the tab 116 is being held and the top layer 130 is being removed.

With continued reference to FIG. 6, an underside 118 of the base layer 110 includes an adhesive 120 configured to adhere to the patient's skin 320. For clarity, only a portion of the underside 118 of the base layer 110 is shown as having the adhesive 120 in FIG. 6. However, in non-limiting embodiments, the adhesive 120 may cover the entire underside 118 of the base layer 110. In non-limiting embodiments, the adhesive 120 may be provided in discrete sections of the underside 118 of the base layer 110. In non-limiting embodiments, the adhesive 120 may be provided in a pattern on the underside 118 of the base layer 110. The adhesive 120 may be a hydrocolloid adhesive, a zinc oxide-based adhesive, and/or any other variety of adhesive suitable for contact with the patient's skin 320. A topside 111 (shown in FIG. 5) of the base layer 110 may be substantially free of an adhesive.

Referring now to FIGS. 1-4, 7, and 8, the top layer 130 is configured to be placed over the base layer 110 and to provide further support for holding the IV treatment line 200 in place relative to the insertion site 310. Further, the top layer 130 may cover the portion of the patient's skin 320 exposed through the cutout 112 of the base layer 110, including the insertion site 310 and a region adjacent to the insertion site 310. The top layer 130 includes or defines a tunnel 132 configured to receive the IV treatment line 200 therethrough. The tunnel 132 may fit around the one or more lumens 220 of the IV treatment line 200 extending from the insertion site 310. The tunnel 132 may include a raised profile offset from the base layer 110 when the top layer 130 is connected to the base layer 110, such that a portion of the top layer 130 surrounding the tunnel 132 may sit flat against the base layer 110 while the IV treatment line 200 passes through the tunnel 132. The tunnel 132 may serve as an alignment feature to assist the clinician in accurately applying the top layer 130 to the base layer 110. In particular, the tunnel 132 may be configured to fit snugly around the IV treatment line 200 such that the top layer 130 must be applied in alignment with the base layer 110 in order for the IV treatment line 200 to be received in the tunnel 132.

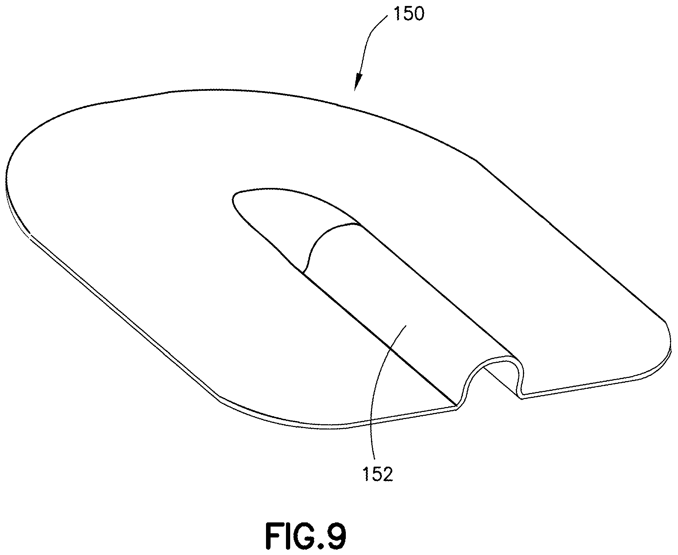

Referring now to FIGS. 1-4 and 9, the top layer 130 includes a window 150 to allow the clinician to observe the insertion site 310 with the top layer 130 in place. The window 150 is transparent or semi-transparent (i.e. at least partially transparent) such that the insertion site 310 can be viewed through the window 150. The position of the window 150 within the top layer 130 corresponds to the position of the cutout 112 in the base layer 110, such that the window 150 overlays the cutout 112 and facilitates observation of the patient's skin 320 exposed by the cutout 112. The window 150 includes a tunnel 152 which is a continuation of the tunnel 132 of the top layer 130. The IV treatment line 200 may extend through the tunnel 152 of the window 150 in the same manner that the IV treatment line 200 extends through the tunnel 132 of the top layer 130. The tunnel 152 terminates at an intermediate location of the window 150, such as a location where a cannula 210 of the IV treatment line 200 is inserted into the insertion site 310. The window 150, at least in the portion thereof corresponding to the cutout 112 of the base layer 110, lacks adhesive such that the window 150 does not bond to the patient's skin 320 exposed by the cutout 112. The window 150 may serve as an alignment feature to assist the clinician in accurately applying the top layer 130 to the base layer 110. In particular, the clinician may observe the cutout 112 of the base layer 110 through the window 150 as the top layer 130 is applied to the base layer 110, and the clinician may place the top layer 130 on the base layer 110 such that the window 150 aligns with the cutout 112.

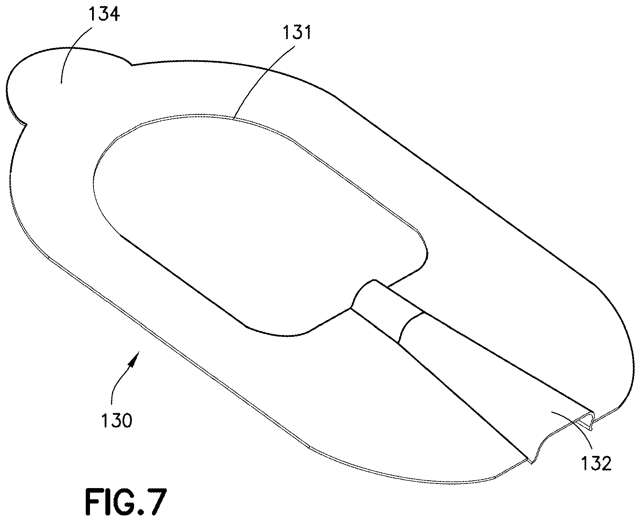

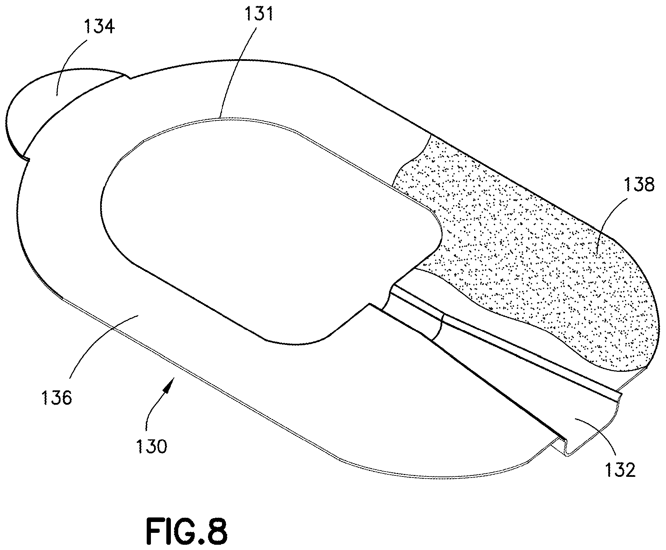

Referring again to FIGS. 7 and 8, the top layer 130 may include an outwardly extending tab 134. In non-limiting embodiments, the tab 134 of the top layer 130 may overlap the tab 116 of the base layer 110 when the dressing cover 100 is assembled. In non-limiting embodiments, the tab 134 of the top layer 130 may be spaced apart from the tab 116 of the base layer 110 when the dressing cover 100 is assembled. A clinician may grasp the tab 134 to remove the top layer 130 from the base layer 110.

Referring now particularly to FIG. 8, an underside 136 of the top layer 130 includes an adhesive 138 configured to releasably hold the top layer 130 to the base layer 110. For clarity, only a portion of the underside 136 of the top layer 130 is shown as having the adhesive 138 in FIG. 8. However, in non-limiting embodiments, the adhesive 138 may cover the entire underside 136 of the top layer 130, with the exception of the window 150. In non-limiting embodiments, the adhesive 138 may be provided in discrete sections of the underside 136 of the top layer 130. In non-limiting embodiments, the adhesive 138 may be provided in a pattern on the underside 136 of the top layer 130. In non-limiting embodiments, the adhesive 138 may not be present in the tunnel 132 so that the top layer 130 does not adhere to the IV treatment line 200. In non-limiting embodiments, the adhesive 138 may not be present on a portion of the underside 136 of the top layer 130 corresponding to the tab 134, such that the tab 134 may be easily grasped. In non-limiting embodiments, the adhesive 138 present on the tab 134 may be weaker than the adhesive 138 on the remainder of the top layer 130, such that the tab 134 may be induced to separate from the base layer 110 under less force than the remainder of the top layer 130.

The adhesive 138 may be a hydrocolloid adhesive, a zinc oxide-based adhesive, a static adhesive, and/or any other variety of adhesive suitable for connecting the top layer 130 to the base layer 110. In non-limiting embodiments, the adhesive 138 of the top layer 130 may be the same type of adhesive as the adhesive 120 of the base layer 110. In non-limiting embodiments, the adhesive 138 of the top layer 130 may be a different type of adhesive as the adhesive 120 of the base layer 110. In non-limiting embodiments, the adhesive 138 of the top layer 130 may be a weaker adhesive than the adhesive 120 of the base layer 110 to prevent base layer from being detached from the patient's skin 320 when the top layer 130 is removed.

In non-limiting embodiments, the adhesive 138 of the top layer 130 may be a stronger adhesive than the adhesive 120 of the base layer 110, and the clinician may hold the tab 116 of the base layer 110 to prevent the base layer 110 from being detached from the patient's skin 320 when the top layer 130 is removed. As the base layer 110 may extend beyond the perimeter of the top layer 130, the adhesive 138 of the top layer 130 typically does not contact the patient.

With continued reference to FIGS. 7 and 8, the top layer 130 may be of substantially any shape. In the non-limiting embodiments shown in the accompanying drawings, the top layer 130 may be four-sided with rounded corners. In non-limiting embodiments, the top layer 130 may be the same shape or substantially the same shape as the base layer 110. In non-limiting embodiments, the top layer 130 may be smaller than the base layer 110 such that the base layer 110 extends beyond the top layer 130 around a perimeter of the top layer 130, thereby preventing the adhesive 138 of the top layer from contacting the patient's skin 320. In non-limiting embodiments, the top layer 130, with the exception of the window 150, may be opaque. In non-limiting embodiments, the top layer 130 inclusive of the window 150 may be at least partially transparent. The top layer 130 defines a cutout 131 for receiving the window 150 during manufacturing of the top layer 130.

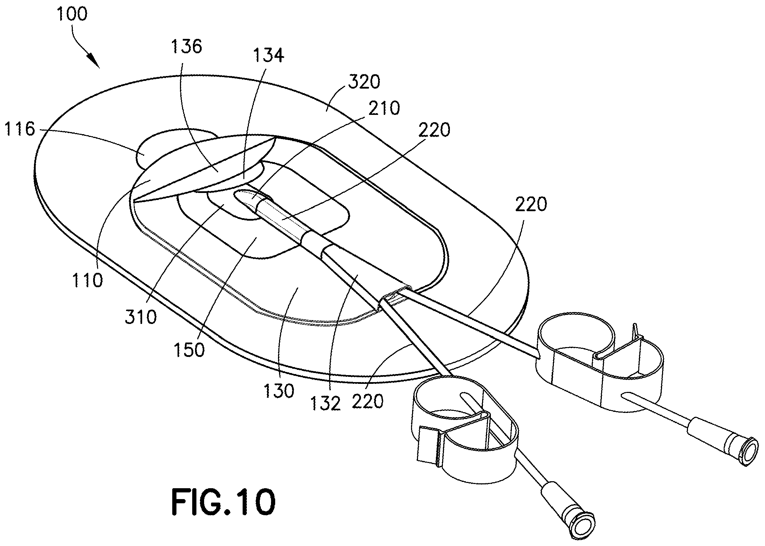

Referring now to FIG. 10, the dressing cover 100 is shown with the top layer 130 partially removed from the base layer 110. The top layer 130 may be removed by grasping the tab 134 and peeling the top layer 130 away from the base layer 110. The clinician may hold the tab 116 of the base layer 110 against the patient's skin 320 during removal of the top layer 130 to prevent the base layer 110 from being removed by the adhesive 138 of the top layer 130. As the top layer 130 is peeled away, the topside 111 of the base layer 110 is exposed. The underside 136 of the top layer 130 is also exposed in the peeled portion of the top layer 130 as the top layer 130 is peeled away.

Referring now to FIG. 11, a force diagram of the dressing cover 100 is shown during removal of the top layer 130. As the top layer 130 is peeled away from the base layer 110, a top layer tension force, illustrated by vector Vt in FIG. 11, is developed in the top layer 130 in a direction in which the top layer 130 is peeled. The top layer tension force may correspond to the force required to separate the adhesive 138 of the top layer 130 from the base layer 110. A base layer tension force, illustrated by vector Vb in FIG. 11, is developed in the base layer 110 in a direction parallel to the patient's skin 320 and in an opposite direction from which the top layer 130 is peeled. Additionally, a stress concentration is present at a point Pk2 of the base layer 110 where the top layer 130 separates from the base layer 110.

Fractions of the top layer tension force and the base layer tension force are imparted to the patient's skin 320, illustrated by vectors Vst and Vsb, respectively, in FIG. 11. Because the base layer 110 remains attached to the patient's skin 320 during removal of the top layer 130, a significant portion of the tension forces produced by removal of the top layer 130 are absorbed by the base layer 110. As such, the magnitudes of the vectors Vst and Vsb, indicative of the force experienced by the patient's skin 320, may be significantly less than the magnitudes of the vectors Vt and Vb. Similarly, the stress concentration at point Pk2 may be substantially absorbed by the base layer 110 rather than being imparted to the patient's skin 320. The more rigid the base layer 110 is, the lower the magnitudes of the vectors Vst and Vsb will be. Thus, a more rigid base layer 110 will reduce the risk of pain and/or skin injury experienced by the patient 300 during removal of the top layer 130 from the base layer 110. Additionally, the adhesive 138 of the top layer 130 can be relatively strong, as the force required to overcome the strength of the adhesive 138 is not fully imparted to the patient's skin 320. Rather, a significant portion of the force required to overcome the strength of the adhesive 138 is absorbed by the base layer 110. In contrast, substantially all of the tension forces developed during removal of a single layer dressing cover are imparted to the patient's skin, as described herein with reference to FIG. 12.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within scope of the appended claims. For example, it is to be understood that the present invention contemplates that, to the extent possible, one or more features of any embodiment can be combined with one or more features of any other embodiment.

## Claims

1. A dressing cover (100) comprising:
a base layer (110) comprising an adhesive (120) configured to adhere to skin (320) of a patient, the base layer (110) defining a cutout (112) configured to provide access to a cannula insertion site (310) of the skin; and
a top layer (130) configured to cover the insertion site (310), the top layer (130) defining a top layer cutout (131), the top layer (130) comprising an adhesive (138) applied to a bottom surface (136) thereof that is removably adhered to the base layer (110);
wherein the top layer (130) includes an at least partially transparent window (150), with the top layer cutout (131) receiving the window (150), and with the window (150) overlaying the cutout (112) of the base layer (110), the window (150) configured to allow observation of the insertion site (310);
**characterized in that** the top layer (130) comprises a tunnel (132) configured to receive a treatment line (200) therethrough and the window (150) comprises another tunnel (152) configured to receive a treatment line (200) therethrough , with the tunnel (152) on the window (150) being a continuation of the tunnel (132) of the top layer (130), and wherein the tunnel (152) on the window (150) terminates at an intermediate portion of the window (150).

2. The dressing cover (100) of claim 1, wherein the base layer (110) extends beyond a perimeter of the top layer (130) and/or wherein the base layer (110) comprises an outwardly extending tab (116).

3. The dressing cover (100) of claim 1, wherein the top layer (130) comprises an outwardly extending tab (134).

4. The dressing cover (100) of claim 3, wherein the adhesive (138) of the top layer (130) is not present on the outwardly extending tab (134) of the top layer (130) or wherein the adhesive (138) of the top layer (130) is weaker on the tab (134) than on a remaining portion of the top layer (130).

5. The dressing cover (100) of claim 3, wherein the base layer (110) comprises an outwardly extending tab (116), and
wherein the outwardly extending tab (134) of the top layer (130) overlaps the outwardly extending tab (116) of the base layer (110).

6. The dressing cover (100) of claim 1, wherein the adhesive (138) of the top layer (130) applied to the bottom surface (136) of the exterior portion is not present on the first portion of the tunnel.

7. The dressing cover (100) of claim 1, wherein the top layer (130) comprises an outwardly extending tab (134) extending from an opposite side of the top layer (130) relative to the tunnel (132, 152).

8. The dressing cover (100) of claim 1, wherein the base layer (110) comprises an adhesive pad (114) configured to adhere to the treatment line (200).

9. The dressing cover (100) of claim 1, wherein the adhesive (138) of the top layer (130) is a static adhesive.

10. The dressing cover (100) of claim 1, wherein the adhesive (138) of the top layer (130) is weaker than the adhesive (120) of the base layer (110) or wherein the adhesive (138) of the top layer (130) is stronger than the adhesive (120) of the base layer (110).

11. A treatment line and dressing cover assembly (100, 200), the assembly comprising:
a dressing cover (100) according to any of claims 1 to 10, and
a treatment line (200) secured between the base layer (110) and the top layer (130), the treatment line (200) comprising a cannula configured for insertion in the insertion site (310).

12. The assembly of claim 11, wherein the base layer (110) extends beyond a perimeter of the top layer (130).

13. The assembly of claim 11, wherein the base layer (110) comprises an adhesive pad (114) configured to adhere to the treatment line (200).

## Patentansprüche

1. Verbandabdeckung (100), die aufweist:
eine Basisschicht (110), die einen Klebstoff (120) aufweist, der dazu dient, an der Haut (320) eines Patienten zu haften, wobei die Basisschicht (110) einen Ausschnitt (112) definiert, der so ausgebildet ist, dass er Zugang zu einer Kanüleneinführstelle (310) der Haut gewährt; und
eine Deckschicht (130), die so ausgebildet ist, dass sie die Einführstelle (310) abdeckt, wobei die Deckschicht (130) einen Deckschicht-Ausschnitt (131) definiert, wobei die Deckschicht (130) einen Klebstoff (138) aufweist, der auf ihre Unterseite (136) aufgebracht ist, die lösbar an der Basisschicht (110) haftet;
wobei die Deckschicht (130) ein zumindest teilweise transparentes Fenster (150) aufweist, wobei der Deckschicht-Ausschnitt (131) das Fenster (150) aufnimmt, und wobei das Fenster (150) den Ausschnitt (112) der Basisschicht (110) überlagert, wobei das Fenster (150) so ausgebildet ist, dass es die Beobachtung der Einführstelle (310) ermöglicht;
**dadurch gekennzeichnet, dass** die Deckschicht (130) einen Tunnel (132) aufweist, der so ausgebildet ist, dass er eine Behandlungsleitung (200) durch sich hindurch aufnimmt, und dass das Fenster (150) einen weiteren Tunnel (152) aufweist, der so ausgebildet ist, dass er eine Behandlungsleitung (200) durch sich hindurch aufnimmt, wobei der Tunnel (152) am Fenster (150) eine Fortsetzung des Tunnels (132) der Deckschicht (130) ist, und wobei der Tunnel (152) am Fenster (150) an einem mittleren Abschnitt des Fensters (150) endet.

2. Verbandabdeckung (100) nach Anspruch 1, wobei sich die Basisschicht (110) über einen Umfang der Deckschicht (130) hinaus erstreckt und/oder wobei die Basisschicht (110) eine nach außen ragende Lasche (116) aufweist.

3. Verbandabdeckung (100) nach Anspruch 1, wobei die Deckschicht (130) eine nach außen ragende Lasche (134) aufweist.

4. Verbandabdeckung (100) nach Anspruch 3, wobei der Klebstoff (138) der Deckschicht (130) nicht auf der nach außen ragenden Lasche (134) der Deckschicht (130) vorhanden ist, oder wobei der Klebstoff (138) der Deckschicht (130) auf der Lasche (134) schwächer ist als auf einem übrigen Abschnitt der Deckschicht (130).

5. Verbandabdeckung (100) nach Anspruch 3, wobei die Basisschicht (110) eine nach außen ragende Lasche (116) aufweist, und
wobei die nach außen ragende Lasche (134) der Deckschicht (130) die nach außen ragende Lasche (116) der Basisschicht (110) überlagert.

6. Verbandabdeckung (100) nach Anspruch 1, wobei der Klebstoff (138) der Deckschicht (130), der auf die Unterseite (136) des äußeren Abschnitts aufgebracht ist, am ersten Abschnitt des Tunnels nicht vorhanden ist.

7. Verbandabdeckung (100) nach Anspruch 1, wobei die Deckschicht (130) eine nach außen ragende Lasche (134) aufweist, die sich von einer entgegengesetzten Seite der Deckschicht (130) relativ zum Tunnel (132, 152) erstreckt.

8. Verbandabdeckung (100) nach Anspruch 1, wobei die Basisschicht (110) ein Klebepad (114) aufweist, das so ausgebildet ist, dass es an der Behandlungsleitung (200) haftet.

9. Verbandabdeckung (100) nach Anspruch 1, wobei der Klebstoff (138) auf der Deckschicht (130) ein statischer Klebstoff ist.

10. Verbandabdeckung (100) nach Anspruch 1, wobei der Klebstoff (138) der Deckschicht (130) schwächer ist als der Klebstoff (120) der Basisschicht (110), oder wobei der Klebstoff (138) der Deckschicht (130) stärker ist als der Klebstoff (120) der Basisschicht (110).

11. Behandlungsleitungs- und Verbandabdeckungsbaugruppe (100, 200), wobei die Baugruppe aufweist:
Verbandabdeckung (100) nach einem der Ansprüche 1 bis 10, und
eine Behandlungsleitung (200), die zwischen der Basisschicht (110) und der Deckschicht (130) befestigt ist, wobei die Behandlungsleitung (200) eine Kanüle aufweist, die zum Einführen in die Einführstelle (310) ausgebildet ist.

12. Baugruppe nach Anspruch 11, wobei sich die Basisschicht (110) über einen Umfang der Deckschicht (130) hinaus erstreckt.

13. Baugruppe nach Anspruch 11, wobei die Basisschicht (110) ein Klebepad (114) aufweist, das so ausgebildet ist, dass es an der Behandlungsleitung (200) haftet.

## Revendications

1. Revêtement de pansement (100) comprenant :
une couche de base (110) comprenant un adhésif (120) configuré pour adhérer à la peau (320) d'un patient, la couche de base (110) définissant une découpe (112) configurée pour fournir un accès à un site d'insertion de canule (310) de la peau ; et
une couche supérieure (130) configurée pour couvrir le site d'insertion (310), la couche supérieure (130) définissant une découpe de couche supérieure (131), la couche supérieure (130) comprenant un adhésif (138) appliqué sur une surface inférieure (136) de celle-ci qui est collé de manière amovible à la couche de base (110) ;
dans lequel la couche supérieure (130) comporte une fenêtre (150) au moins partiellement transparente, la découpe (131) de couche supérieure recevant la fenêtre (150), et la fenêtre (150) recouvrant la découpe (112) de la couche de base (110), la fenêtre (150) étant configurée pour permettre l'observation du site d'insertion (310) ;
**caractérisé en ce que** la couche supérieure (130) comprend un tunnel (132) configuré pour recevoir une ligne de traitement (200) à travers celui-ci et la fenêtre (150) comprend un autre tunnel (152) configuré pour recevoir une ligne de traitement (200) à travers celui-ci, le tunnel (152) sur la fenêtre (150) étant une continuation du tunnel (132) de la couche supérieure (130), et dans lequel le tunnel (152) sur la fenêtre (150) se termine au niveau d'une partie intermédiaire de la fenêtre (150).

2. Revêtement de pansement (100) selon la revendication 1, dans lequel la couche de base (110) s'étend au-delà d'un périmètre de la couche supérieure (130) et/ou dans lequel la couche de base (110) comprend une languette (116) s'étendant vers l'extérieur.

3. Revêtement de pansement (100) selon la revendication 1, dans lequel la couche supérieure (130) comprend une languette (134) s'étendant vers l'extérieur.

4. Revêtement de pansement (100) selon la revendication 3, dans lequel l'adhésif (138) de la couche supérieure (130) n'est pas présent sur la languette (134) de la couche supérieure (130) s'étendant vers l'extérieur ou dans lequel l'adhésif (138) de la couche supérieure (130) est plus faible sur la languette (134) que sur une partie restante de la couche supérieure (130).

5. Revêtement de pansement (100) selon la revendication 3, dans lequel la couche de base (110) comprend une languette (116) s'étendant vers l'extérieur, et
dans lequel la languette (134) de la couche supérieure (130) s'étendant vers l'extérieur chevauche la languette (116) de la couche de base (110) s'étendant vers l'extérieur.

6. Revêtement de pansement (100) selon la revendication 1, dans lequel l'adhésif (138) de la couche supérieure (130) appliqué sur la surface inférieure (136) de la partie extérieure n'est pas présent sur la première partie du tunnel.

7. Revêtement de pansement (100) selon la revendication 1, dans lequel la couche supérieure (130) comprend une languette (134) s'étendant vers l'extérieur s'étendant depuis un côté opposé de la couche supérieure (130) par rapport au tunnel (132, 152).

8. Revêtement de pansement (100) selon la revendication 1, dans lequel la couche de base (110) comprend un tampon adhésif (114) configuré pour adhérer à la ligne de traitement (200).

9. Revêtement de pansement (100) selon la revendication 1, dans lequel l'adhésif (138) de la couche supérieure (130) est un adhésif statique.

10. Revêtement de pansement (100) selon la revendication 1, dans lequel l'adhésif (138) de la couche supérieure (130) est plus faible que l'adhésif (120) de la couche de base (110) ou dans lequel l'adhésif (138) de la couche supérieure (130) est plus fort que l'adhésif (120) de la couche de base (110).

11. Ensemble de ligne de traitement et de revêtement de pansement (100, 200), l'ensemble comprenant :
un revêtement de pansement (100) selon l'une quelconque des revendications 1 à 10, et
une ligne de traitement (200) fixée entre la couche de base (110) et la couche supérieure (130), la ligne de traitement (200) comprenant une canule configurée pour être insérée dans le site d'insertion (310).

12. Ensemble selon la revendication 11, dans lequel la couche de base (110) s'étend au-delà d'un périmètre de la couche supérieure (130).

13. Ensemble selon la revendication 11, dans lequel la couche de base (110) comprend un tampon adhésif (114) configuré pour adhérer à la ligne de traitement (200).
